# EUROPÄISCHE PATENTSCHRIFT

(11) **EP 0 915 082 B1**
(45) Veröffentlichungstag und Bekanntmachung des Hinweises auf die Patenterteilung: **19.12.2001**
(21) Anmeldenummer: 98119418.6
(22) Anmeldetag: 14.10.1998
(51) Int. Cl.: C07C 277/08, C07C 279/14

(54) **Verfahren zur Herstellung von substituierten Guanidinderivaten**
Process for the preparation of substituted guanidine derivatives
Procédé pour la préparation de derivés de guanidine substitués

(30) Priorität: 04.11.1997 DE 19748697
(43) Veröffentlichungstag der Anmeldung: 12.05.1999
(73) Patentinhaber: BASF AKTIENGESELLSCHAFT, 67056 Ludwigshafen (DE)
(72) Erfinder: Greindl, Thomas Dr., 67098 Bad Dürkheim (DE); Scherr, Günter Dr., 67065 Ludwigshafen (DE); Schneider, Rolf Dr., 68163 Mannheim (DE); Mundinger, Klaus Dr., 67117 Limburgerhof (DE)

(56) Entgegenhaltungen:
- EP-A- 0 754 679
- DE-C- 964 590
- P.E. GAGNON ET AL: CAN. J. CHEM., Bd. 36, 1958, Seiten 737-743, XP002069686

## Beschreibung

Die vorliegende Erfindung betrifft ein verbessertes Verfahren zur Herstellung von substituierten Guanidiniumverbindungen durch Umsetzungen von Calciumcyanamid mit einer primären oder sekundären Aminocarbonsäure oder einer primären oder sekundären Aminosulfonsäure oder deren Derivate.

Substituierte Guanidiniumverbindungen sind in der Natur weit verbreitet. Wichtige Vertreter dieser Substanzklasse sind beispielsweise Aminosäuren, wie Arginin und Kreatin. Darüberhinaus sind substituierte Guanidinverbindungen als sterisch gehinderte Basen, als Biozide sowie als Komplexliganden bekannt. Die Mehrzahl der Verbindungen dieses Typs sind aufgrund der hohen Herstellkosten in ihrer technischen Anwendbarkeit jedoch stark eingeschränkt.

Ein Beispiel für ein biologisch aktives Guanidinderivat ist Kreatin, das als "Energieträger der Zelle" zur Nahrungsergänzung im Food und Pharmabereich eingesetzt wird.

Die Synthese von Guanidiniumsalzen aus Cyanamid ist bekannt und wird beispielhaft beschrieben in US 2,425,341, wo wässrige Cyanamid Lösungen bei Temperaturen >80°C mit wässrigen Lösungen der Amine bei pH-Werten > 8 umgesetzt werden.

Die Herstellung von Kreatin wird z.B. in EP-A-0 754 679 und der dort zitierten weiterführenden Literatur beschrieben, wobei maximale Ausbeuten von lediglich 70% erzielt werden.

Ein Nachteil der o.g. Synthesen für Guanidiniumverbindungen ist die Verwendung wässriger Lösungen von reinem Cyanamid. Diese Lösungen sind sehr teuer und aufgrund der Instabilität des Cyanamids im allgemeinen nicht breit verfügbar.

Die großtechnische Herstellung von reinem Cyanamid oder wäßrigen Lösungen davon erfolgt ausgehend von Kalkstickstoff. Die Produktion ist jedoch aufwendig, da wegen der großen Instabilität von Cyanamid bei Herstellung und Lagerung besondere Vorsichtmaßnahmen getroffen werden müssen. Die Herstellung ist beispielhaft beschrieben in D.R.P. 267514 und D.R.P. 648542.

Es bestand daher die Aufgabe, ein kostengünstiges und einfach durchführbares Verfahren zur Herstellung substituierter Guanidine auf Basis breit verfügbarer Einsatzstoffe bereitzustellen, das die oben genannten Nachteile nicht aufweist.

Diese Aufgabe wurde gelöst durch ein Verfahren zur Herstellung von substituierten Guanidinderivaten der Formel I, 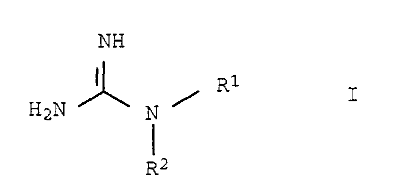 dadurch gekennzeichnet, daß man Calciumcyanamid mit einer primären oder sekundären Aminocarbonsäure oder einer primären oder sekundären Aminosulfonsäure oder deren Derivate der allgemeinen Formel II, 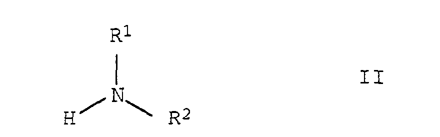 umsetzt, wobei die Substituenten R¹ und R² unabhängig voneinander folgende Bedeutung haben:
- R¹: H,
C₁-C₂₀-Alkyl;
- R²: ―(C₁-C₂₀-Alkylen)―COOR³, ―(C₁-C₂₀-Alkylen)―CONR⁴R⁵, ―(C₁-C₂₀-Alkylen)―CN, ―(C₁-C₂₀-Alkylen)―SO₂R⁶;
- R³: H, C₁-C₂₀-Alkyl, C₂-C₁₀-Alkenyl, C₃-C₈-Cycloalkyl, C₆-C₁₈-Aryl, Na, K, Li, Ca, Mg, N(R¹)₄;
- R⁴ und R⁵: unabhängig voneinander
H, C₁-C₂₀-Alkyl, C₂-C₁₀-Alkenyl, C₃-C₈-Cycloalkyl, C₆-C₁₈-Aryl;
- R⁶: OR⁷, N(R⁸)₂
- R⁷: H, C₁-C₂₀-Alkyl, C₂-C₁₀-Alkenyl, C₃-C₈-Cycloalkyl, C₆-C₁₈-Aryl, Na, K, Li, Ca, Mg, N(R¹)₄;
- R⁸: H, C₁-C₂₀-Alkyl, C₂-C₁₀-Alkenyl, C₃-C₈-Cycloalkyl, C₆-C₁₈-Aryl.

Das erfindungsgemäße Verfahren erfüllt insbesondere wirtschaftliche Randbedingungen, wie niedrige Einsatzstoffkosten, technisch einfache Durchführbarkeit, verbesserte Ausbeuten und hinreichende Reinheit des Produktes.

Geeignet sind für die Umsetzung mit Calciumcyanamid prinzipiell alle beanspruchten Aminocarbonsäuren und Aminosulfonsäuren und deren Derivate der Formel II.

Als Alkylreste für R¹, R³ bis R⁵ sowie für R⁷ und R⁸ seien verzweigte oder unverzweigte C₁-C₂₀-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, l-Ethyl-2-methylpropyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als Alkenylreste für R³ bis R⁵ sowie für R⁷ und R⁸ seien verzweigte oder unverzweigte C₂-C₁₀-Alkenylketten, bevorzugt Vinyl, Propenyl, Isopropenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 2-Hexenyl, 1-Heptenyl, 2-Heptenyl, 1-Octenyl oder 2-Octenyl genannt.

Als Alkylenreste für R² seien verzweigte oder unverzweigte C₁-C₂₀-Alkylenketten, bevorzugt Methylen, Ethylen, n-Propylen, 1-Methylethylen, n-Butylen, 1-Methylpropylen-, 2-Methylpropylen, 1,1-Dimethylethylen, n-Pentylen, 1-Methylbutylen, 2-Methylbutylen, 3-Methylbutylen, 2,2-Dimethylpropylen, 1-Ethylpropylen, n-Hexylen, 1,1-Dimethylpropylen, 1,2-Dimethylpropylen, 1-Methylpentylen, 2-Methylpentylen, 3-Methylpentylen, 4-Methylpentylen, 1,1-Dimethylbutylen, 1,2-Dimethylbutylen, 1,3-Dimethylbutylen, 2,2-Dimethylbutylen, 2,3-Dimethylbutylen, 3,3-Dimethylbutylen, 1-Ethylbutylen, 2-Ethylbutylen, 1,1,2-Trimethylpropylen, 1,2,2-Trimethylpropylen, 1-Ethyl-1-methylpropylen, 1-Ethyl-2-methylpropylen, n-Heptylen, n-Octylen, n-Nonylen, n-Decylen, n-Undecylen, n-Dodecylen, n-Tridecylen, n-Tetradecylen, n-Pentadecylen, n-Hexadecylen, n-Heptadecylen, n-Octadecylen, n-Nonadecylen oder n-Eicosylen genannt.

Die 1- bis 20-gliedrigen Alkylenketten können optional mit folgenden Resten substituiert sein:
C₁-C₆-Alkyl, beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, l-Ethyl-l-methylpropyl, 1-Ethyl-2-methylpropyl;
Sulfhydrylmethyl, 1-Aminobutyl, 1-Carboxyethyl;
Arylalkyl, beispielsweise Benzyl, p-Hydroxybenzyl, Indolylmethyl.

Als Cycloalkylreste seien für R³ bis R⁵ sowie für R⁷ und R⁸ bevorzugt verzweigte oder unverzweigte C₃-C₈-Cycloalkylreste wie Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1-Pentylcyclopropyl, 1-Methyl-1-Butylcyclopropyl, 1,2-Dimethylcyclopropyl, 1-Methyl-2-Ethylcyclopropyl oder Cyclooctyl genannt.

Die Cycloalkylreste können ggf. mit einem oder mehreren, z.B. 1 bis 3 Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein oder 1 bis 3 Heteroatome wie Schwefel, Stickstoff, dessen freie Valenzen durch Wasserstoff oder C₁-C₄-Alkyl abgesättigt sein können oder Sauerstoff im Ring enthalten.

Als Alkoxyreste für R⁶ kommen solche mit 1 bis 20 C-Atomen, vorzugsweise mit 1 bis 12 C-Atomen, besonders bevorzugt 1 bis 8 C-Atomen in Betracht.

Beispielsweise sind zu nennen:
- Methoxy: Ethoxy-
- Isopropoxy-: n-Propoxy-
- 1-Methylpropoxy-: n-Butoxy-
- n-Pentoxy-: 2-Methylpropoxy-
- 3-Methylbutoxy-: 1,1-Dimethylpropoxy-
- 2,2-Dimethylpropoxy-: Hexoxy-
- 1-Methyl-1-ethylpropoxy-: Heptoxy-
- Octoxy-: 2-Ethylhexoxy-

Als mono- oder disubstituierte Aminoreste für R⁶ kommen bevorzugt solche in Betracht, die Alkylreste mit 1 bis 20, bevorzugt 1 bis 12 C-Atomen enthalten, wie z.B. Methyl-, n-Propyl-, n-Butyl-, 2-Methylpropyl-, 1,1-Dimethylpropyl-, Hexyl-, Heptyl-, 2-Ethylhexyl-, Isopropyl-, 1-Methylpropyl-, n-Pentyl-, 3-Methylbutyl-, 2,2-Dimethylpropyl-, 1-Methyl-1-ethylpropyl- und Octyl.

Als Tetraalkylammoniumreste für R³ und R⁷ kommen solche in Betracht, die Alkylreste mit 1 bis 20, bevorzugt 1 bis 12, besonders bevorzugt 1 bis 6 C-Atomen enthalten, wie z.B. Methyl-, n-Propyl-, Isopropyl-, 2-Methylpropyl-, 1,1-Dimethylpropyl-, 1-Methylpropyl-, 2,2-Dimethylpropyl-, 1-Methyl-1-ethylpropyl-, n-Butyl-, 3-Methylbutyl-, n-Pentyl- und Hexyl-.

Unter Aryl sind aromatische Ringe oder Ringsysteme mit 6 bis 18 Kohlenstoffatomen im Ringsystem zu verstehen, beispielsweise Phenyl oder Naphthyl, die ggf. mit einem oder mehreren Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein können. Bevorzugt sind ggf. substituiertes Phenyl, Methoxyphenyl und Naphthyl.

Als bevorzugt verwendete Verbindungen der Formel II seinen alle die in Wasser oder in, mit Wasser mischbaren Lösungsmitteln löslichen, primären und sekundären Aminocarbonsäuren oder Aminosulfonsäuren genannt. Besonders bevorzugte Vertreter sind u.a. Taurin und Aminocarbonsäuren wie Glycin, Alanin, Valin, Prolin, Leucin, Phenylalanin, Lysin, Methionin, Cystein, Asparaginsäure, Iminodiessigsäure, Sarkosin sowie deren Ester, Amide und Nitrile und deren Salze.

Ganz besonders bevorzugte Verbindung der Formel II ist Sarkosin, das sowohl als freie Säure als auch insbesondere als Na- oder K-Salz in Form einer 5 bis 60 Gew.-%igen, bevorzugt 35 bis 45 Gew.-%igen wäßrigen Lösung verwendet werden kann.

Das erfindungsgemäße Verfahren zur Herstellung von substituierten Guanidinderivaten zeichnet such besonders dadurch aus, daß anstelle des teueren reinen Cyanamids, welches üblicherweise als kristallines Reinprodukt, oder als eine bei pH 3 bis 6 stabilisierte Lösung im Handel ist, sehr billiger und breit verfügbarer Kalkstickstoff eingesetzt werden kann.

Unter Kalkstickstoff versteht man Produkte, die z.B. durch Umsetzung von CaC₂ mit N₂ bei sehr hohen Temperaturen von 800 - 1100 °C entstehen. Sie enthalten in der Regel 5 bis 98 Gew.-%, bevorzugt 20 bis 95 Gew.-%, besonders bevorzugt 30 bis 90 Gew.-% Calciumcyanamid. Der technisch erhältliche graue bis schwarze Kalkstickstoff enthält neben Calciumcyanamid zusätzlich noch Verunreinigungen, wie z.B. Kohlenstoff, Ca-Carbid, CaO sowie Spuren von Metallen üblicherweise in Anteilen <1%. Es kann selbstverständlich auch reines Ca-Cyanamid eingesetzt werden. Besonders vorteilhaft, weil wirtschaftlicher, ist jedoch der Einsatz von technischem, nicht hochreinem Kalkstickstoff. Dieser wird vorzugsweise pulverförmig, in einer Korngrößenverteilung von 1 bis 100 µm, eingesetzt. Es kann aber auch gekörntes, gestrangtes, oder anderweitig verdichtetes Material, als auch eine entsprechende Aufschlämmung in Wasser, Alkoholen oder anderen wassermischbaren Lösungsmitteln eingesetzt werden.

Die Umsetzung von Calciumcyanamid mit Ammoniak ist in US 2,114,280 beschrieben. Hierbei wird Calciumcyanamid mit einem Überschuß an Ammoniak unter Druck und Temperaturen >130°C zu unsubstituiertem Guanidin umgesetzt.

Gagnon et al. in Can. J. Chem. 36 (1958) beschreiben die Umsetzung von Calciumcyanamid mit primären C₁-C₅-Amin-Nitraten.

Wie von H. Michaud et al. in Chem. Ztg. 112 (1988) 10, 287-294 und von F. Baum in Biochem. Z. 26 (1910), 325-332 beschrieben, entstehen bei der Hydrolyse von Kalkstickstoff ohne Säurezugabe, teils große Mengen an Nebenprodukten, wie z.B. Dicyandiamid und Harnstoff.

Zur Vermeidung dieser Nebenreaktionen muß zum einen die Temperatur unter 40°C, besser unter 20°C gehalten werden, was aufwendige Kühlung notwendig macht, zum anderen muß durch Zugabe von Säuren, insbesondere von Kohlensäure der pH-Wert in den sauren Bereich gebracht werden.

Diese Prozedur ist jedoch zeitaufwendig und liefert zudem eine stark verdünnte Lösung an Cyanamid, die durch zusätzliche Abdestillation aufkonzentriert werden muß.

Es ist weiterhin bekannt, daß die Hydrolyse von Cyanamid im Alkalischen durch Schwermetallionen, insbesondere durch Eisen und Mangan, katalysiert wird.

Deshalb war es umso überraschender, daß das technische Ca-Cyanamid mitsamt Schwermetallspuren im Alkalischen in guten Ausbeuten zum Guanidin-Derivat umsetzbar ist.

Ein weiterer Vorteil des Verfahrens besteht in der Tatsache, daß der verwendete Kalkstickstoff, der selbst in Wasser alkalisch reagiert, nicht in einem getrennten Schritt sauer hydrolysiert werden muß, sondern überraschenderweise ohne weitere Vorbehandlung direkt beim angestrebten pH-Wert für die Umsetzung mit dem Amin eingesetzt werden kann. Üblicherweise wird dabei ein pH-Wert im Bereich des pK-Wertes des Amins verwendet, d.h. in einem Bereich zwischen 6 und 14, bevorzugt zwischen 8 und 12, besonders bevorzugt im pH-Bereich von 9 bis 11.

Dies spart, verglichen mit der getrennten Herstellung von Cyanamid aus Calciumcyanamid und der weiteren Umsetzung zum Guanidin beträchtliche Säuremengen, da im erfindungsgemäßen Verfahren ausschließlich im Alkalischen gearbeitet wird.

Die Synthese kann in der Art und Weise erfolgen, daß man Kalkstickstoff zu einer Lösung eines primären oder sekundären Amins unter Konstanthaltung des o.g. pH-Bereichs zudosiert. Dabei kann die Zudosierung in fester Form oder als Suspension erfolgen. Die Reaktionstemperatur liegt dabei im Bereich von 20 bis 120°C, insbesondere im Bereich von 40 bis 80°C.

Die Zugabe von Kalkstickstoff erfolgt in gleichmäßigen Portionen über einen Zeitraum von 0,5 bis 10 Std., bevorzugt von 1 bis 6 Std., besonders bevorzugt über einen Zeitraum von 2,5 bis 3 Std.

Nach der Dosierung wird in der Regel 0,5 bis 10 Std., bevorzugt 1 bis 3 Std. nachgerührt.

Das Molverhältnis von Calciumcyanamid zu primärem oder sekundärem Amin liegt im Bereich von 0,9 bis 5,0, bevorzugt von 0,9 bis 4,0, insbesondere im Bereich von 1,0 bis 2,0.

Zur Entfernung gelöster Schwermetallionen kann es vorteilhaft sein, Komplexbildner wie Phosphate, Sulfate, Aminopolycarboxylate, beispielsweise EDTA oder Aminopolyphosphonate einzusetzen.

Zur Beseitigung geruchsbildender Nebenprodukte können zusätzlich Oxidationsmittel, wie z.B. H₂O₂ zugesetzt werden.

Auf diese Weise kann die Reinheit des gebildeten Guanidinderivates verbessert werden, ohne daß es zu Ausbeuteverlusten kommt.

Nach erfolgter Umsetzung lassen sich eventuell ausgefallene anorganische Nebenprodukte bei Temperaturen von 20 bis 100°C, bevorzugt 50 bis 90°C, nach an sich bekannten Verfahren, wie z.B. Filtration oder Zentrifugation abtrennen.

Die Isolierung der gewünschten Guanidiniumderivate erfolgt in an sich bekannter Weise. So läßt sich beispielsweise durch Abkühlen der abfiltrierten Reaktionslösung auf -20 bis 60°C, insbesondere 0 bis 40°C, das Wertprodukt kristallin erhalten. Nach Filtration kann gegebenenfalls durch eine weitere Umkristallisation die Reinheit verbessert werden. Es ist aber auch möglich, daß Produkt mittels Extraktion aus dem Reaktionsgemisch zu entfernen, um es anschließend durch Destillation oder Kristallisation sauber zu isolieren.

Ein weiterer Vorteil des erfindungsgemäßen Verfahrens liegt darin, daß man auf eine technisch aufwendige Kühlung, wie sie bei der Herstellung von Cyanamid benötigt wird, verzichten kann. Aufgrund der angewendeten milden Temperaturen kann die Umsetzung, technisch besonders einfach, drucklos erfolgen.

Es ist in besonderer Weise überraschend, daß die Ausbeuten der erfindungsgemäßen Reaktion mit denen der Umsetzung von reinem Cyanamid vergleichbar sind, und deshalb, bezogen auf den Ca-Cyanamid-Gehalt für den Gesamtprozeß von Freisetzung des Cyanamids und Umsetzung zum Guanidiniumsalz aufgrund der geringeren Zahl an Verfahrensschritten höher liegt.

Zudem können noch höhere Umsätze erzielt werden, wenn man den billigen Kalkstickstoff im Überschuß einsetzt, was bei Einsatz von reinem Cyanamid häufig unwirtschaftlich ist. Die erreichte Reinheit des isolierten Guanidiniumsalzes ist mit der aus reinem Cyanamid hergestellten Ware vergleichbar. Dies ist insbesondere überraschend, da es sich um einen technischen stark verunreinigten Einsatzstoff handelt.

Als Einsatzstoffe können neben dem Ca-Salz des Cyanamids auch andere Erdalkalisalze herangezogen werden.

Auch im Falle der verwendeten primären und sekundären Amine können hier neben den reinen Stoffen vorteilhafterweise vor allem technische Produkte eingesetzt werden, wie z.B. anstelle von reinem Sarkosin eine technische Na-Salz-Lösung mit Wirkstoff-Gehalten von 5 - 60%, bevorzugt aber 40%, die gegebenenfalls auch noch wasserlösliche Verunreinigungen enthalten können, wie z.B. N-Methyl-iminodiessigsäure.

Der Einsatz von technischen Produkten empfiehlt sich insbesondere wenn keine weiteren reaktiven Amine beigemischt sind und es aus wirtschaftlichen Gründen besonders vorteilhaft ist, weil z.B. die Aufreinigung des Amins teuer und verlustreich ist.

Zur Einhaltung des pH-Wertes können je nach Ausgangs-pH-Wert der Base entweder Säuren, wie CO₂, SO₂, HCl, HNO₃, H₂SO₄, H₂SO₃, H₃PO₃, H₃PO₂ und H₃PO₄, und/oder Basen wie NaOH, KOH, LiOH, Ca(OH)₂, Ba(OH)₂, Mg(OH)₂ eingesetzt werden. Sollten die Amine in basischer und nicht in neutralisierter oder teilneutralisierter Form vorliegen, so werden nur Säuren benötigt.

Als Säuren sind solche bevorzugt, die technisch leicht verfügbar sind und zusammen mit Schwermetallspuren zu schwerlöslichen Komplexen führen, wie z.B. CO₂, H₂SO₄, H₃PO₄. Es können aber auch bevorzugt Gemische dieser und anderer Säuren eingesetzt werden.

In den folgenden Beispielen wird das Verfahren zur Herstellung substituierter Guanidiniumderivate näher erläutert.

### Beispiel 1

### Umsetzung von Sarkosin-Na-Salz-Lösung mit Ca-Cyanamid bei pH 10 in Gegenwart von Schwefelsäure

In einem 21-Rührreaktor mit Rückflußkühler, Blattrührer, Thermostat und pH-gesteuerter Schwefelsäuredosierung wurden zu einem Gemisch von 133 g 40,1%iger wässriger Na-Sarkosinat-Lösung und 53 g Wasser zur Einstellung eines pH-Wertes von 10 insgesamt 14,0 g 50%ige Schwefelsäure zudosiert. Anschließend wurden bei einer Temperatur von 60°C innerhalb von 2 Std. portionsweise insgesamt 87,2 g Kalkstickstoff (Fa. Aldrich, gekörntes Produkt, CaCN₂-Gehalt = 84%) zugegeben. Um den pH-Wert von 10 zu halten wurden über den Zugabezeitraum insgesamt 403 g 25%ige Schwefelsäure zudosiert. Die Reaktionstemperatur wurde durch Kühlung bei 60°C gehalten. Nach beendeter Zugabe wurde noch 2 Std. bei der angegebenen Temperatur weitergerührt, und anschließend innerhalb von 20 min auf 80°C erhitzt. Die entstandene grünliche Suspension wurde bei 80°C filtriert und der schwarze Filterkuchen 2 mal mit 250 ml 80°C warmem Wasser gewaschen. Mutterlauge und Waschwasser wurden vereinigt und mit 2 g 85%iger H₃PO₄ behandelt. Der sich bildende feine farblose Niederschlag wurde erneut abfiltriert und die nun farblose Lösung auf 5°C abgekühlt. Nach Abtrennung der gebildeten Kristalle wurde die verbleibende Mutterlauge im Vakuum auf ca. 50% Restmenge eingeengt wobei nach Erkalten eine 2.Kristallfraktion anfiel. Insgesamt wurden nach Trocknung 66,4 g farbloses Kristallisat erhalten, mit einem Kreatin-Gehalt von 89,0% und einem Restwassergehalt von 11%, entsprechend einer isolierten Ausbeute von 58%. Der Gehalt an Kreatin in der Mutterlauge lag bei 1,3%, damit errechnet sich eine Ausbeute der Reaktion von 68%.

### Beispiel 2

### Umsetzung von Sarkosin-Na-Salz-Lösung mit Ca-Cyanamid bei pH 11 in Gegenwart von Schwefelsäure

Die, mit Ausnahme des pH-Wertes (pH 11 statt pH 10) analog Beispiel 1 durchgeführte Reaktion lieferte Kreatin in einer Gesamtausbeute von 56%.

### Beispiel 3

### Umsetzung von Sarkosin-Na-Salz-Lösung mit Ca-Cyanamid bei pH 10 in Gegenwart von Phosphorsäure

Die Reaktion wurde analog Beispiel 1 durchgeführt. Die pH-Wert Einstellung erfolgte mittels 85%iger Phosphorsäure. Man erhielt Kreatin mit einer Gesamtausbeute von 75% und einer Reinheit vor der Umkristallisation von ca. 89%.

### Beispiel 4

### Umsetzung von Sarkosin-Na-Salz-Lösung mit Ca-Cyanamid bei pH 10 in Gegenwart einer Mischung aus Schwefelsäure und Phosphorsäure

In der analog Beispiel 1 durchgeführten Reaktion wurde zur pH-Wert Einstellung anstelle von Schwefelsäure ein Gemisch aus Schwefelsäure und Phosphorsäure verwendet. Man erhielt Kreatin mit einer Gesamtausbeute von 86% und einer Reinheit vor der Umkristallisation von ca. 95%.

## Patentansprüche

1. Verfahren zur Herstellung von substituierten Guanidinderivaten der Formel I, 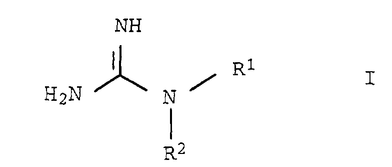 **dadurch gekennzeichnet, daß** man Calciumcyanamid mit einer primären oder sekundären Aminocarbonsäure oder einer primären oder sekundären Aminosulfonsäure oder deren Derivate der allgemeinen Formel II, 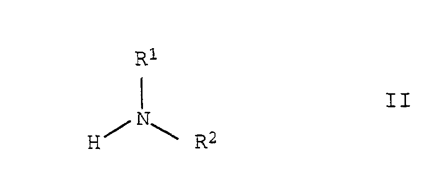 umsetzt, wobei die Substituenten R¹ und R² unabhängig voneinander folgende Bedeutung haben:
R¹ H,
C₁-C₂₀-Alkyl;
R² ―(C₁-C₂₀-Alkylen)―COOR³, ―(C₁-C₂₀-Alkylen)―CONR⁴R⁵, ―(C₁―C₂₀―Alkylen)―CN, ―(C₁-C₂₀-Alkylen)―SO₂R⁶;
R³ H, C₁-C₂₀-Alkyl, C₂-C₁₀-Alkenyl, C₃-C₈-Cycloalkyl, C₆-C₁₈-Aryl,
Na, K, Li, Ca, Mg, N(R¹)₄;
R⁴ und R⁵ unabhängig voneinander
H, C₁-C₂₀-Alkyl, C₂-C₁₀-Alkenyl, C₃-C₈-Cycloalkyl, C₆-C₁₈-Aryl;
R⁶ OR⁷, N(R⁸)₂;
R⁷ H, C₁-C₂₀-Alkyl, C₂-C₁₀-Alkenyl, C₃-C₈-Cycloalkyl, C₆-C₁₈-Aryl,
Na, K, Li, Ca, Mg, N(R¹)₄;
R⁸ H, C₁-C₂₀-Alkyl, C₂-C₁₀-Alkenyl, C₃-C₈-Cycloalkyl, C₆-C₁₈-Aryl.

2. Verfahren nach Anspruch 1, **dadurch gekennzeichnet, daß** man Calciumcyanamid mit einem primären oder sekundären Amin der Formel II in Wasser und/oder einem mit Wasser mischbaren organischen Lösungsmittel bei einer Temperatur von 20 bis 120°C und einem pH-Wert von 6 bis 14 umsetzt.

3. Verfahren nach den Ansprüchen 1 und 2, **dadurch gekennzeichnet, daß** man Calciumcyanamid in Form von technischem Kalkstickstoff mit einem Gehalt an Calciumcyanamid von 30 bis 95 Gew.-% verwendet.

4. Verfahren nach den Ansprüchen 1 bis 3, **dadurch gekennzeichnet, daß** man eine wasserlösliche oder eine in einem wassermischbaren Lösungsmittel lösliche Aminocarbonsäure oder Aminosulfonsäure oder deren Derivate verwendet.

5. Verfahren nach Anspruch 4, **dadurch gekennzeichnet, daß** man Sarkosin als Aminocarbonsäure verwendet.

## Claims

1. A process for preparing substituted guanidine derivatives of the formula I, 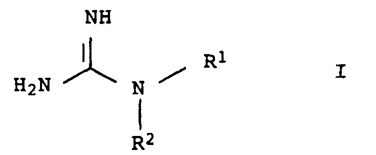 which comprises reacting calcium cyanamide with a primary or secondary amino carboxylic acid or a primary or secondary amino sulfonic acid or derivatives thereof, of the formula II 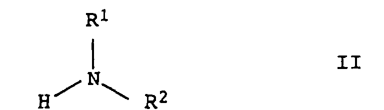 where the substituents R¹ and R² have, independently of one another, the following meanings:
R¹ H,
C₁-C₂₀-alkyl;
R² ―(C₁-C₂₀-alkylene)―COOR³,
―(C₁-C₂₀-alkylene)―CONR⁴R⁵, ―( C₁-C₂₀-alkylene)―CN,
―(C₁-C₂₀-alkylene)―SO₂R⁶;
R³ H, C₁-C₂₀-alkyl, C₂-C₁₀-alkenyl, C₃-C₈-cycloalkyl, C₆-C₁₈-aryl,
Na, K, Li, Ca, Mg, N(R¹)₄;
R⁴ and R⁵ independently of one another
H, C₁-C₂₀-alkyl, C₂-C₁₀-alkenyl, C₃-C₈-cycloalkyl, C₆-C₁₈-aryl;
R⁶ OR⁷, N(R⁸)₂;
R⁷ H, C₁-C₂₀-alkyl, C₂-C₁₀-alkenyl, C₃-C₈-cycloalkyl, C₆-C₁₈-aryl,
Na, K, Li, Ca, Mg, N(R¹)₄;
R⁸ H, C₁-C₂₀-alkyl, C₂-C₁₀-alkenyl, C₃-C₈-cycloalkyl, C₆-C₁₈-aryl.

2. A process as claimed in claim 1, wherein calcium cyanamide is reacted with a primary or secondary amine of the formula II in water and/or a water-miscible organic solvent at from 20 to 120°C and at a pH of from 6 to 14.

3. A process as claimed in claims 1 and 2, wherein calcium cyanamide is used in the form of technical nitrolime with a calcium cyanamide content of from 30 to 95% by weight.

4. A process as claimed in claims 1 to 3, wherein the amino carboxylic acid or amino sulfonic acid or derivatives thereof used is/are soluble in water or in a water-miscible solvent.

5. A process as claimed in claim 4, wherein sarcosine is used as amino carboxylic acid.

## Revendications

1. Procédé pour la préparation de dérivés substitués de la guanidine répondant à la formule I 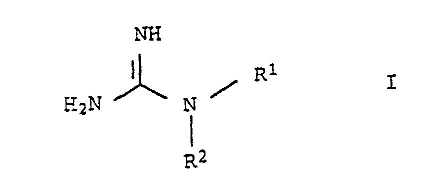 **caractérisé par le fait que** l'on fait réagir le cyanamide calcique avec un acide aminocarboxylique primaire ou secondaire ou avec un acide aminosulfonique primaire ou secondaire ou leurs dérivés répondant à la formule générale II 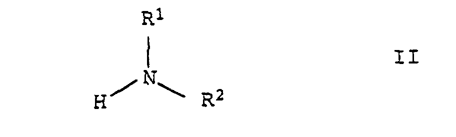 dans laquelle les symboles R¹ et R² représentent chacun, indépendamment l'un de l'autre :
R¹ : H,
un groupe alkyle en C1-C20 ;
R² : un groupe -(alkylène en C1-C20)-COOR³, -(alkylène en C1-C20)-CONR⁴R⁵, -(alkylène en C1-C20)-CN, -(alkylène en C1-C20)-SO₂R⁶ ;
R³ : H, un groupe alkyle en C1-C20, alcényle en C2-C10, cycloalkyle en C3-C8, aryle en C6-C18,
Na, K, Li, Ca, Mg, N(R¹)₄ ;
R⁴ et R⁵ représentent chacun, indépendamment l'un de l'autre : H, un groupe alkyle en C1-C20, alcényle en C2-C10, cycloalkyle en C3-C8, aryle en C6-C18 ;
R⁶ : OR⁷, N(R⁸)₂;
R⁷ : H, un groupe alkyle en C1-C20, alcényle en C2-C10, cycloalkyle en C3-C8, aryle en C6-C18,
Na, K, Li, Ca, Mg, N(R¹)₄;
R⁸ : H, un groupe alkyle en C1-C20, alcényle en C2-C10, cycloalkyle en C3-C8, aryle en C6-C18.

2. Procédé selon la revendication 1, **caractérisé par le fait que** l'on fait réagir le cyanamide calcique avec une amine primaire ou secondaire de formule II dans l'eau et/ou dans un solvant organique miscible à l'eau à une température de 20 à 120°C et un pH de 6 à 14.

3. Procédé selon les revendications 1 et 2, **caractérisé par le fait que** l'on utilise le cyanamide calcique à l'état de cyanamide calcique technique à une teneur réelle en cyanamide calcique de 30 à 95 % en poids.

4. Procédé selon les revendications 1 à 3, **caractérisé par le fait que** l'on utilise en acide aminocarboxylique ou un acide aminosulfonique ou leurs dérivés solubles dans l'eau ou solubles dans un solvant miscible à l'eau.

5. Procédé selon la revendication 4, **caractérisé par le fait que** l'acide aminocarboxylique utilisé est la sarcosine.
